# EUROPÄISCHE PATENTSCHRIFT

(11) **EP 0 801 055 B1**
(45) Veröffentlichungstag und Bekanntmachung des Hinweises auf die Patenterteilung: **05.01.2000**
(21) Anmeldenummer: 97105178.4
(22) Anmeldetag: 27.03.1997
(51) Int. Cl.: C07C 265/10, C08G 18/75

(54) **Homologen- und Isomerengemische von cycloaliphatischen Diisocyanaten sowie ihre Verwendung als Aufbaukomponente bei der Herstellung von Polyisocyanat-Additionsprodukten**
Mixtures of homologues and isomers of cycloaliphatic diisocyanates and their use in the preparation of polyisocyanate addition products
Mélanges d'homologues et d'isomères de diisocyanates cycloaliphatiques ainsi que leur utilisation dans la préparation de produits d'addition de polyisocyanates

(30) Priorität: 11.04.1996 DE 19614270
(43) Veröffentlichungstag der Anmeldung: 15.10.1997
(73) Patentinhaber: BAYER AG, 51368 Leverkusen (DE)
(72) Erfinder: Scholl, Hans-Joachim, Dr., 51061 Köln (DE); Jansen, Bernhard, Dr., 51061 Köln (DE); Jost, Klaus, Dr., 41539 Dormagen (DE); Meyer, Rolf-Volker, Dr., 51373 Leverkusen (DE)

(56) Entgegenhaltungen:
- EP-A- 0 058 368
- EP-A- 0 128 382
- US-A- 3 351 650

## Beschreibung

Die vorliegende Erfindung betrifft neue Homologen- und Isomerengemische von cycloaliphatischen Diisocyanaten, sowie ihre Verwendung bei der Herstellung von Polyisocyanat-Additionsprodukten. Cycloaliphatische Diisocyanate sind beispielsweise aus US-A-3351650 bekannt. Ein übliches Verfahren verläuft über die Kernhydrierung entsprechender aromatischer Diamine und nachfolgender Phosgenierung der so erhaltenen cycloaliphatischen Diamine. So lassen sich z.B. Methyl-cyclohexandiamin-lsomerengemische durch Kernhydrierung aus Diaminotoluol herstellen und anschließend zum entsprechenden Methylcyclohexandiisocyanat-Isomerengemisch phosgenieren. Nachteilig bei diesem bekannten Verfahren sind auf der Hydrierstufe a) Desaminierungsreaktionen, bedingt durch die drastischen Hydrierbedingungen, die ausbeutemindernd wirken und als Folgeprodukte störende Monoisocyanatanteile verursachen und b) der prozentuale große Anteil cis-ständiger Diamino-Isomerer, die aufgrund ihrer engen Nachbarschaft in unerwünscht hohem Anteil Nebenreaktionen intramolekularer Art eingehen können. So bilden diese im Zuge konventioneller Phosgenierverfahren zwischenzeitlich intramolekulare Harnstoffbrücken aus, die nachfolgend biuretisieren und somit zu drastisch reduzierten Isocyanatausbeuten beitragen.

Es war daher Aufgabe der vorliegenden Erfindung, Cyclohexandiisocyanat-Derivate zur Verfügung zu stellen, welche nach einem einfachen Verfahren zugänglich sind und welche die oben aufgezeigten Nachteile im beschriebenen Ausmaß nicht aufweisen. Diese Aufgabe konnte mit dem nachstehend näher beschriebenen erfindungsgemäßen Verfahren gelöst werden.

Gegenstand der Erfindung sind Homologen- und Isomerengemische von Diisocyanaten der nachfolgenden Formel (1) 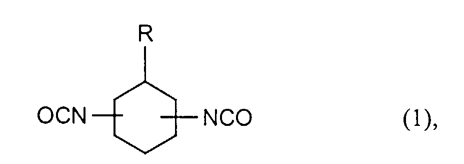 in welcher
- R: für einen gesättigten, geradkettigen, aliphatischen Kohlenwasserstoffrest mit 8 bis 15 Kohlenstoffatomen steht
und welche durch Kernhydrierung zu Verbindungen der nachfolgenden Formel (2) 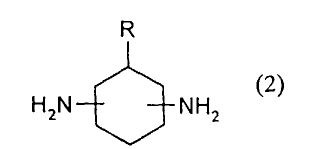 und anschließende Phosgenierung der so erhaltenen Aminogruppen ausgehend von als Homologen- und Isomerengemisch vorliegenden aromatischen Diaminen der nachfolgenden Formel (3) 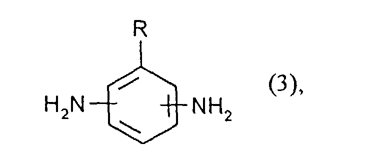 erhalten werden. R besitzt in Formel (2) und (3) die gleiche Bedeutung wie in Formel (1).

Gegenstand der Erfindung ist auch die Verwendung der neuen Diisocyanatgemische bei der Herstellung von Polyisocyanat-Additionsprodukten.

Ausgangsmaterialien für das erfindungsgemäße Verfahren sind die gemäß EP 0 058 335 herstellbaren Diamine 3. Durch die Verwendung dieser erfindungswesentlichen Ausgangsprodukte, bestehend aus einer Vielzahl von Homologen und Isomeren und bedingt durch die langen, linearen Alkylketten, werden die erfindungsgemäßen n-C₈-C₁₅-Alkyl-cyclohexandiisocyanate 1 in einfacher Art durch Kernhydrierung und anschließende Phosgenierung erhalten, ohne merkliche Nebenproukte vom Typ "Monoisocyanat" und mit vergleichsweise vermindertem Anteil an Isocyanatgruppen verbrauchenden Nebenprodukten vom Typ "Biuret", herrührend von cis-ständigen Diamino-Isomeren. Dieser erfindungswesentliche Befund ist als äußerst überraschend anzusehen.

Die erste Stufe des erfindungsgemäßen Verfahrens besteht in einer an sich bekannten Kernhydrierung, d.h. einer katalytisch ausgelösten Hydrierung des aromatischen Kerns. Als Katalysatoren werden übliche, für die Kernhydrierung geeignete Verbindungen eingesetzt.

Die bei dieser Kernhydrierung anfallenden Diamine der Formel 2 entsprechen selbstverständlich bezüglich der Stellung der Aminogruppen den für das erfindungswesentliche Ausgangsmaterial 3 gemachten Angaben.

Zur Herstellung der erfindungsgemäßen Diisocyanatgemische werden die so erhaltenen als Homologen- und Isomerengemische vorliegenden Diamine einer an sich bekannten Phosgenierungsreaktion unterzogen. Hierzu wird beispielsweise das Diamin in einem Hilfslösungsmittel wie Chlorbenzol gelöst und in eine Lösung von Phosgen in Chlorbenzol unter Rühren und Kühlen bei -10 bis 0°C eingetropft (Kaltphosgenierung), wonach das Reaktionsgemisch unter fortlaufendem Rühren und Einleiten von Phosgen langsam bis zum Rückfluß erhitzt wird, um das zunächst gebildete Carbamidsäurechlorid in das angestrebte Diisocyanat zu überführen (Heißphosgenierung). Anschließend wird das Reaktionsgemisch in an sich bekannter Weise aufgearbeitet. Selbstverständlich kann die Überführung der Diaimine in die erfindungsgenmäßen Diisocyanate auch nach beliebigen, anderen an sich bekannten Phosgeniermethoden erfolgen, z,.B. nach Lösungsmittel-freien Phosgenierverfahren oder durch Phosgenierung der Diamine in der Gasphase.

Die erfindungsgemäßen Diisocyanate stellen bei Raumtemperatur flüssige Substanzen dar, die keinerlei störende Anteile an Monoisocyanaten enthalten. Eventuell verfahrensbedingte geringe Anteile an Biuret lassen sich leicht destillativ abtrennen. Die undestillierten Typen, vorzugsweise aber die Destillate, stellen daher wertvolle Kettenverlängerungsmittel bei der Herstellung von Polyisocyanat-Additionsprodukten dar.

### Beispiele

### Beispiel A (Herstellung einer aminischen Ausgangsverbindung)

In diesem Beispiel wird ein als Homologen- und Isomerengemisch vorliegendes aromatisches Diamin gemäß EP 00 58 335 eingesetzt, deren Alkylketten eine Länge von 10 bis 13 C-Atomen bei einer mittleren Kettenlänge von etwa 12 C-Atomen aufweisen.

In einem 3l-Rührautoklaven werden 776 g Diamin, 763 g tert.-Butanol und 7,7 g Rutheniumoxidhydrat vorgelegt. Der Autoklav wird dreimal mit Stickstoff gespült. Anschließend werden 138 bar Wasserstoff aufgedrückt, der Ansatz unter Rühren auf 180°C erhitzt und das eingesetzte aromatische Diamin im Druckbereich von 270 bis 259 bar kernhydriert. Nach einer Reaktionszeit von 5 Stunden ist die Wasserstoffaufnahme beendet. Nach Entspannen des Autoklaven und Abtrennen des Katalysators wird das Filtrat am Rotationasverdampfer eingeengt und das Rohamin nachfolgend unter vermindertem Druck destilliert. Auf diese Weise können 739 g Diamingemisch als bei 130 bis 152°C / 0,1 mbar siedende Fraktion erhalten werden (Ausbeute: 95 %).

### Beispiel 1 (Herstellungsbeispiel eines erfindungsgemäßen Diisocyanats)

In diesem Beispiel wird ein als Homologen- und Isomerengemisch vorliegendes Diamin gemäß Beispiel A eingesetzt, deren Alkylketten eine Länge von 10 bis 13 C-Atomen bei einer mittleren Kettenlänge von etwa 12 C-Atomen aufweisen.

In einem 4-l Vierhalskolben mit Rührer, Thermometer, Gaseinleitungsrohr und Rückflußkühler versehen, werden 2 l trockenes Chlorbenzol vorgelegt. Unter Rühren und Kühlung (-10°C) werden 500 g Phosgen einkondensiert. Anschließend läßt man unter Kühlung bei -10 bis -5°C 370 g Diamin, in 300 g Chlorbenzol gelöst, zutropfen. Unter weiterem Einleiten von Phosgen erwärmt man langesam bis auf Rückflußtemperaturen. Nach Ende der Chlorwasserstoffentwicklung wird das überschüssige Phosgen mit Stickstoff ausgeblasen und die Lösung im Vakuum eingedampft. Man erhält 345 g Rohisocyanatgemisch mit einem NCO-Gehalt von 19,1 % (Theorie: 25,1 %), das nach IR-spektroskopischen Untersuchungen geringe Biuret-Anteile enthält. Das so erhaltene erfindungsgemäße Rohprodukt kann ohne weitere Reinigung als Ausgangsmaterial zur Herstellung von Polyurethanen verwendet werden, oder aber einer zusätzlichen destillativen Reinigung unterworfen werden. 300 g des Rohprodukts werden unter vermindertem Druck destilliert. Bei einem Druck von 0,5 mbar destillieren innerhalb des Temperaturbereichs von 150 bis 175°C 225 g eines praktisch farblosen Gemischs von Diisocyanten der Formel 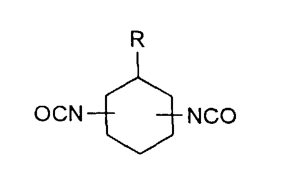 in der R obige Bedeutung hat.

| Analyse (%) | NCO | C | H | N |
|---|---|---|---|---|
| gefunden: | 25,0 | 71,7 | 10,4 | 8,2 |
| Theorie: (bezogen auf C₂₀H₃₄N₂O₂) | 25,1 | 71,9 | 10,2 | 8,4 |

Nach Kernresonanzmessungen sind die Isocyanatgruppen des Diisocyanats transständig in meta-Stellung zueinander angeordnet.

### Verwendungsbeispiele

### Beispiel 2 (Herstellung der wasserdispergierbaren Isocyanate als Leimungsmittel; erfindungsgemäß)

85 g des destillierten Isocyanates aus Beispiel 1 werden bei 60°C vorgelegt und 15 g eines auf Ethylenglykolmonomethylether gestarteten Polyethers mit einem mittleren Molekulargewicht von 350 g/mol unter Rühren zugegeben. Es wird gerührt bis der Isocyanatgehalt 20,4 % beträgt.

Das Präparat ist eine lagerstabile wasserhelle Flüssigkeit, welche sich sowohl zu einem als auch zu fünf Gewichtsteilen sehr gut in Wasser dispergieren läßt.

### Beispiel 3 (Leimung von Papier)

Papier mit einem Flächengewicht von 80 g/m² wird auf einer Labor-Leimpresse da. Fa. Mathis, Zürich, Schweiz, Typ HF mit einer wäßrigen Dispersion des Isocyanates aus Beispiel 2 behandelt. Die Flotten enthielten außer den in Tabelle 2 beschriebenen Leimungsmittel noch 5 % Stärke (Perfectamyl der Fa. AVEBE; Niederlande). Die so ausgerüsteten Papiere werden durch Abpressen mit Filz entwässert und dann 10 Minuten bei 90°C im Trockenschrank getrocknet.

Die Leimungswirkung wird durch den Cobb-Test geprüft. Hierbei wird nach DIN 53 132 die einseitige Wasseraufnahme eines Papiers innerhalb 60 Sek. gravimetrisch ermittelt. Der gefundene Wert ist eine Maßzahl für den Leimungsgrad; die Werte sind in der folgenden Tabelle zusammengefaßt.

| Leimungsmittel | Einsatzmenge (% Wirksubstanz) | Cobb-Wert |
|---|---|---|
| Aquapel 2B∗ | 0,15 | 22,4 |
| Baysynthol KSN-W∗∗ | 0,15 | 27,9 |
| Produkt aus Bsp. 2 | 0,15 | 21,4 |

| | | |
|---|---|---|
| ∗ Produkt der Fa. Hercules, enthält ca. 12 % Wirksubstanz (Vergleich) | | |
| ∗∗ Prdoukt der Fa. Bayer AG, enthält ca. 21,4 % Wirksubstanz (Vergleich) | | |

### Beispiel 4 (Herstellung eines in Alkohol/Testbenzin löslichen Polyurethan-Harnstoffs auf Basis des erfindungsgemäßen Diisocyanats gemäß Beispiel 1 und des Diamins gemäß Beispiel A)

70 g eines Polyesterdiols aus Basis Adipinsäure/Hexandiol-1,6/Neopentylglykol (1:0,71:0,45 molar), OHZ: 66 und 24,7 g eines Polyesterdiols auf Basis Phthalsäureanhydrid/Ethylenglykol OHZ: 281 werden entwässert, mit 100 g des erfindungsgemäßen Diisocyanats gemäß Beispiel 1 vermischt und in zwei Stunden bei 100 bis 105°C zu einem Semiprepolymer mit einem NCO-Gehalt von 8,3 % umgesetzt. Man verdünnt mit 337 g Testbenzin (Kp 155 bis 185), kühlt auf ca. 15°C und fügt 208 g Isopropanol hinzu. In die klare Lösung wird nachfolgend bei 15 bis 25°C eine Lösung aus 54,3 g Diamin gemäß Beispiel A, 167 g Isopropanol und 40 g Methoxypropanol-2 so eingetropft, daß die Amin-Kettenverlängerung zügig vonstatten geht. Das Zutropfen wird kurz vor dem Äquivalenzpunkt abgebrochen (IR-Kontrolle: die Lösung weist nur noch eine minimale NCO-Bande auf). Danach waren 98 % der zuzutropfenden Lösung verbraucht. Man erhält eine dünnflüssige, klare Lösung mit einem Feststoffanteil an 25 % und einer Viskosität (25°C) von 33 mPas. Ein entsprechend hergestelltes Produkt mit 50 % Feststoffanteil stellt ebenfalls eine klare Lösung dar mit einer Viskosität (25°C) von 17 000 mPas.

### Beispiel 5 (Vergleichsbeispiel)

Wird die Herstellung einer Lösung mit 25 % Feststoffanteil gemäß Beispiel 4 unter äquimolarem Ersatz des dort verwendeten Diisocyanats und Diamins durch die technisch bekannten cycloaliphatischen Analoga "Isophorondiisocyanat" und "Isophorondiamin" (Fa. Merck) versucht, so sind in keiner Phase des Herstellungsprozesses klare Lösungen zu erzwingen: die trübe Flüssigkeit wird im Zuge der Kettenverlängerung zunehmend inhomogen, sodaß die Kettenverlängerung aufgrund der Ausbildung eines zweiphasigen Systems nach Zugabe von 80 % der aminischen Lösung abgebrochen wurde.

Die Beispiele 4 und 5 belegen, daß nur unter Verwendung des erfindungsgemäßen Diisocyanats klare Lösungen unter Mitverwendung von Testbenzin erhältlich sind. Dies stellt insofern einen technischen Vorteil dar, weil bisher anstelle von Testbenzin toxikologisch bedenkliche Lösungsmittel wie z.B. Toluol oder Dimethylformamid mitverwendet werden mußten, um brauchbare, klare Lösungen zu erhalten.

## Patentansprüche

1. Homologen- und Isomerengemische von Diisocyanaten der Formel (1) 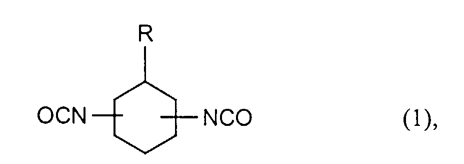 in welcher
R für einen gesättigten, geradkettigen, aliphatischen Kohlenwasserstoffrest mit 8 bis 15 Kohlenstoffatomen steht.

2. Verfahren zur Herstellung von Verbindungen gemäß Anspruch 1, dadurch gekennzeichnet, daß als Homologen- und Isomerengemisch vorliegenden aromatische Diamine der Formel (3) 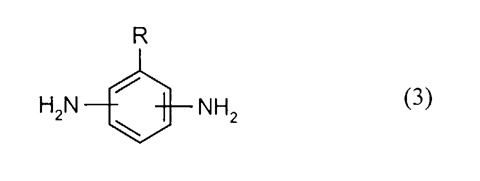 durch Kernhydrierung zu Verbindungen der Formel (2) 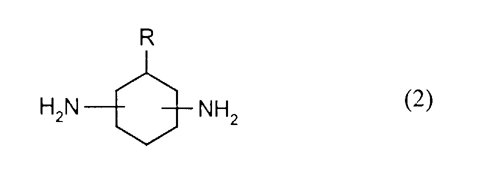 hergestellt und anschließend die Aminogruppen dieser Verbindung phosgeniert werden.

3. Verwendung der Homologen- und Isomerengemische gemäß Anspruch 1 bei der Herstellung von Polyisocyanat-Additionsprodukten.

## Claims

1. Homologue and isomer mixtures of diisocyanates of the formula (1) 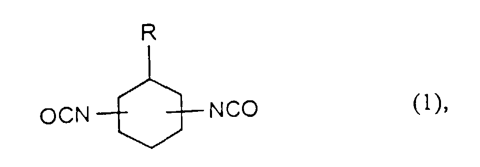 in which
R denotes a saturated, linear, aliphatic hydrocarbon residue having 8 to 15 carbon atoms.

2. Process for the production of compounds according to claim 1, characterised in that aromatic diamines of the formula (3) present as a homologue and isomer mixture 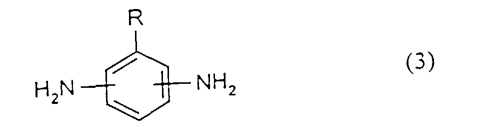 are produced by ring hydrogenation to yield compounds of the formula (2) 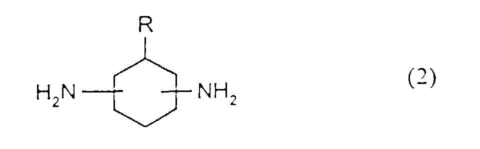 and the amino groups of this compound are then phosgenated.

3. Use of the homologue and isomer mixtures according to claim 1 in the production of polyisocyanate addition products.

## Revendications

1. Mélanges d'homologues et d'isomères de diisocyanates répondant à la formule (1) 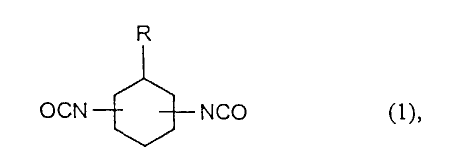 dans laquelle
R représente un radical d'hydrocarbure saturé, à chaîne droite, aliphatique, contenant de 8 à 15 atomes de carbone.

2. Procédé pour la préparation de composés selon la revendication 1, caractérisé en ce qu'on prépare des diamines aromatiques présentes sous la forme d'un mélange d'homologues et d'isomères, répondant à la formule (3) 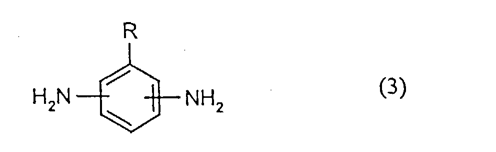 par hydrogénation du noyau pour obtenir des composés répondant à la formule (2) 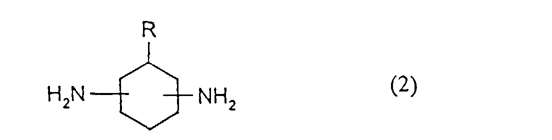 et ensuite, on soumet les groupes amino de ce composé à une phosgénation.

3. Utilisation des mélanges d'homologues et d'isomères selon la revendication 1 lors de la préparation de produits d'addition de polyisocyanates.
